# EUROPEAN PATENT SPECIFICATION

(11) **EP 1 360 939 B1**
(45) Date of publication and mention of the grant of the patent: **23.01.2008**
(21) Application number: 03010505.0
(22) Date of filing: 09.05.2003
(51) Int. Cl.: A61B 17/86

(54) **Screw for fixing atlantoaxial joint**
Schraube zum Ruhigstellen des Atlantoaxialgelenks
Vis pour l'immobilisation de l'articulation atlanto-axial

(30) Priority: 09.05.2002 JP 2002134261
(43) Date of publication of application: 12.11.2003
(73) Proprietor: Showa IKA Kohgyo Co., Ltd., Aichi-ken (JP)
(72) Inventor: Oribe, Kazuya, c/o Showa Ika kohgyo Co., Ltd., Tokyo (JP); Takamido, Hiroshi, c/o Showa Ika kohgyo Co., Ltd., Nagoya-shi, Aichi-ken (JP)
(74) Representative: Grünecker, Kinkeldey, Stockmair & Schwanhäusser Anwaltssozietät

(56) References cited:
- EP-A- 0 856 293
- DE-A- 19 755 369
- DE-U- 29 703 947
- US-A- 5 743 914
- US-A- 6 030 162
- US-A1- 2001 021 852
- PATENT ABSTRACTS OF JAPAN vol. 1998, no. 13, 30 November 1998 (1998-11-30) -& JP 10 211213 A (MITSUBISHI MATERIALS CORP), 11 August 1998 (1998-08-11)
- DATABASE WPI Section Ch, Week 199432 Derwent Publications Ltd., London, GB; Class D22, AN 1994-261654 XP002262065 -& RU 2 005 432 C (DOKTOR IMPLANTS CENTRE) , 15 January 1994 (1994-01-15)

## Description

### BACKGROUND OF THE INVENTION

### Field of the Invention

The present invention relates to screws for fixing atlantoaxial joints used for spondylodesis, a medical treatment for spondylous patients such as atlantoaxial joint instability.

### Description of the Related Art

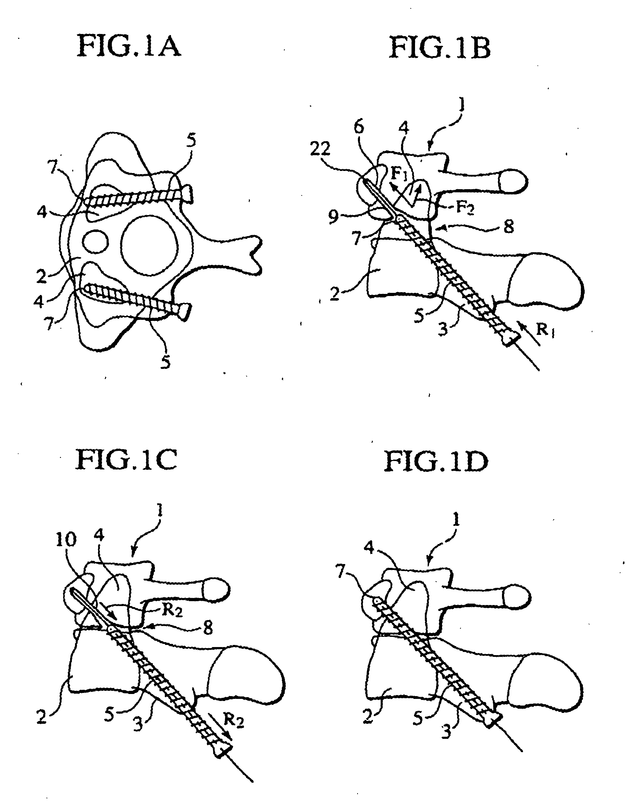

As shown in Figs.1A to 1D, as an example of a method for fixing an atlantoaxial joint of an atlas (the first cervical vertebra) and an axis (the second cervical vertebra), there is a method in which a pair of right and left screws 5 are screwed and embedded into lateral masses 4 of an atlas 1 from inferior articular processes 3 of an axis 2.

More specifically, after little holes 22 are bored in advance at the positions where the screws 5 are to be screwed and embedded into the first and the second cervical vertebrae to the axis 2 and the atlas 1, the screws 5 are screwed and embedded in the direction of R₁ in Fig. 1B. Ends 7 of the screws 5 used in this example have flat shapes, i.e. shapes which are cut close to perpendicular to a longitudinal direction of the screws 5. Therefore, an attachment of the screws 5 to the atlas 1 and the axis 2 is bad, and particularly when the screws 5 are screwed into a joint 8 where the atlas 1 and the axis 2 are lapped over, there has been problems such as that the screws 5 are not screwed into articular surface holes 9 of the atlas 1, the end faces in the traveling direction of the screws 5 abut the atlas 1, the atlas 1 is pushed away from the axis 2 by component force F₂ of pressing force F₁ of the screws 5 (Fig. 1B).

Therefore, when the screws 5 are screwed and fixed, actions has been taken in such a way that, after formation of embedding parts 10 where the ends of the screws 5 are slightly embedded in the articular surface holes 9 of the atlas 1 is confirmed, the screws 5 are temporarily drawn back in the direction of an arrow R₂ in Fig. 1C. Thereafter, when an upper part of the atlas 1 is returned to the original position by its own weight (Fig. 1 C), the screws 5 are threaded again.

In the above method, the screws 5 should be pulled back and inserted, resulting in bad operating efficacy.

DE 19755369 describes an apparatus for fixing the first and second cervical vertebrae, consisting of a screw, provided with a blunt tip and two tapper flutes, and a thread portion extending over the entire length of the screw. The screw is fastened into an area between the two vertebrae such that the vertebrae are fixed. The dimensions of the screw are such that the screw engages both vertebrae but does not project out of the area between the vertebrae. Additionally, a clamping device can be provided for respectively joining the vertebrae.

DE 29703947 Ul describes an apparatus for percutaneously screwing an articulation, in particular for blocking and/or fusion of a vertebral articulation. It is provided with a first wire, the tip of which can percutaneously be positioned on the processus articularis superior of a vertebra, a second wire, the tip of which percutaneously can be positioned on the processus costalis of the vertebra, and a sleeve adjustable relative to the connection line between the tips of the wires for guiding of a bone drill and positioning of a bone screw.

US 20010021852 depicts a cervical anchor having an elongated body with a proximal and a distal end, a passage extending inside the body from the proximal end, and at least one hole extending at least partially readily through the body that communicates with the passage. The passage and the (at least) one hole are configured so that a material is receivable in the passage, preferably at the proximal end of the elongated body. So configured, the material may be delivered through the passage, through the (at least) one hole and into the skeletal member.

US 5743914 describes a bone screw having a head with a socket for receiving a screw driving device and a shaft extending from the head. The shaft includes respectively alternating first and second helical threads running substantially parallel with each other along the shaft. Additionally, the second series of helical threads exhibits a diameter substantially different from the first series of the helical threads. In addition, the bone screw may comprise a plurality of cervical threads, each exhibiting different diameters from the others. The screw may have a through hole, and a cutting portion on its tip, and is suitable for general bone use. The preamble of claim 1 is based on this screw. JP-A-10211213 discloses screws with a pair of protrusions to facilitate insertion.

### SUMMARY OF THE INVENTION

The invention is devised in consideration of the conventional problems as described above. According to the present invention, there is provided a screw for fixing an atlantoaxial joint comprising, a shaft body having a through-hole in a center of the shaft body from one end to the other end, a screw part provided on one end of the shaft body, and a cutting portion for screwing is provided on the one tip end of the screw part wherein the cutting portion comprises a pair of acute protrusions having cutter blades which are formed at crossing portions between inclined planes and taper faces at the end of the shaft body.

### BRIEF DESCRIPTION OF THE DRAWINGS

Figs, 1A to 1D are explanatory drawings illustrating a conventional example of a method for fixing atlantoaxial joints.
Figs. 2A to 2C are explanatory drawings illustrating a usage example of screws for fixing atlantoaxial joints according to the invention.
Figs. 3A to 3C are explanatory drawings for the screws for fixing atlantoaxial joints according to the invention.

### DETAILED DESCRIPTION OF THE INVENTION

An embodiment of the present invention will be described below based on accompanying drawings.

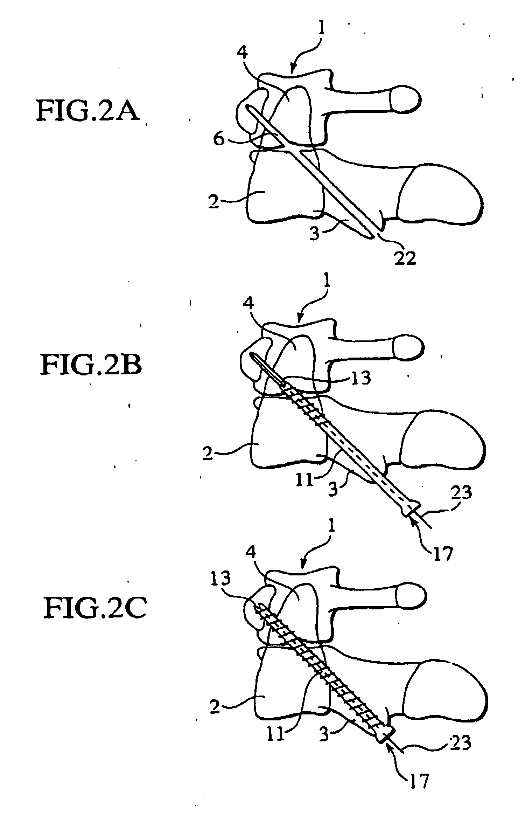

As shown in Figs. 2A to 2C, a screw for fixing operation of an atlantoaxial joint 11 (hereinafter referred to as "screw 11") used for the fusion of an atlantoaxial joint of an atlas 1 and an axis 2 is diagonally screwed and embedded into a lateral mass 4 of the atlas 1 from an inferior articular process 3 of the axis 2 as the conventional screws 5 (refer to Fig. 1A).

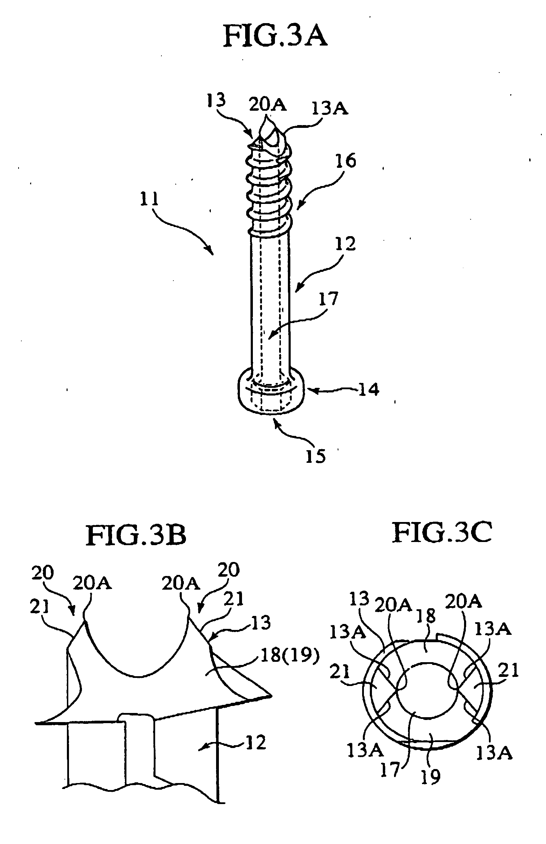

As shown in Fig. 3A, the screw 11 has a cutter portion 13 provided on one end side of a shaft body 12, and a head with a large diameter 14 having a tool engaging hole 15, which is provided on the other end side of the shaft body 12.

On one end side of the shaft body 12, a screw 16 is provided with the cutter portion 13. In the center of the shaft body 12, a through-hole 17 is provided from the head 14 to the cutter portion 13.

As shown in Figs. 3B and 3C, the cutter portion 13 has a pair of facing protrusions 20 provided by forming the first inclined plane 18 and the second inclined plane 19 in approximately symmetric figure at the end of the shaft body 12. The clearance between inclined planes 18, 19 is getting small from the shaft body 12 to a tip portion thereof. Furthermore, on the outer peripheral surface on the end side of the protrusions 20, taper faces 21 whose end sides are oriented inside are formed to acutely form ends 20A of the protrusions 20.

In other words, cutter blades 13A are formed at crossing portions of the inclined planes 18, 19 and the taper faces 21.

A method of fixing cervical vertebrae by using the screw 11 is described with reference to Figs. 2A to 2C as below:

In order to link and fix the axis 2 and the lateral mass 4 by the screw 11, firstly, a hole 22 is opened diagonally to the cervical vertebrae by a boring tool such as an air drill and the like from the inferior articular process 3 of the axis 2 to the lateral mass 4 of the atlas 1, and secondly, a guiding line 23 such as a wire (not shown), a guide pin (not shown) and the like is inserted in the through-hole 17 of the screw 11, and then the screw 11 is screwed into the hole 22 by using the guiding line 23 as a guide. In other words, the guiding line 23 is used as a guide by inserting it through the through-hole 17 of the screw 11.

When the screw 11 is screwed into a hole 6 which is bored in the lateral mass 4 of the atlas 1 from the inferior articular process 3 of the axis 2, as the screw 11 is pressured and fixed along with the rotation thereof, the cutter blades 13A of the cutting portion 13 are screwed toward inside over the scraping inside of the hole 22.

As the ends 20A of the both protrusions 20 can be elastically deformed so that they can close each other due to component forces which act inward on the cutter blades 13A in cutting, screwing into the hole 22 becomes easy.

According to the embodiment, at the start of the screwing, an end diameter of the screw 11 in the direction of screwing becomes small due to the component forces, so that screwing into the target is easy to perform. In the course of screwing, the screw 16 is surely embedded in the vertebrae due to reaction force which makes the diameter return to the original length against the component forces. By further continuing to screw the screw 16, the atlantoaxial joint can be easily fixed.

Additionally, a trend that the screw 11 pushes away the atlas 1 in screwing can be restrained, so that the conventional problem as mentioned above can be solved.

Therefore, as the atlas 1 is never spaced from the axis 2 by the screw 11, there is no need to take conventional actions such as that the screw 5 embedded in a embedding part 10 of the atlas 1 is temporarily backed up, and the screw 5 is screwed again after the atlas 1 returns to the original position, so that efficiency can be improved.

This Application claims Priority of a Japanese Application No. P2002-134261 with a filing date of May 9, 2002.

Although the invention has been described above by reference to certain embodiments of the present invention, the invention is not limited to the embodiments described above and will occur to those skilled in the art, in light of the teachings. The scope of the invention is defined with reference to the following claims.

## Claims

1. A screw for fixing an atlantoaxial joint (11) comprising:
a shaft body (12) having a through-hole (17) in a center of the shaft body (12) from one end to the other end;
a screw part (16) provided on one end of the shaft body (12); and
a cutting portion (13) for screwing on the tip of the screwing part (16)
**characterized in that,**
the cutting portion (13) comprises a pair of acute protrusions (20) having cutter blades (13a) which are formed at crossing portions between inclined planes (18, 19) and taper faces (21) at the end of the shaft body (12).

2. A screw (11) for fixing an atlantoaxial joint according to claim 1, wherein:
the pair of acute protrusions (20) face each other and are formed by cutting the tip of the screw (16) in a state of inclination, and the protrusions comprise the cutting blades (13A).

3. A screw (11) for fixing an atlantoaxial joint according to claim 1, wherein:
the shaft body (12) comprises a tool-engaging hole (15).

## Patentansprüche

1. Schraube zum Fixieren eines Atlantoaxialgelenks (11), die umfasst:
einen Schaftkörper (12) mit einem Durchgangsloch (17) in einer Mitte des Schaftkörpers (12) von einem Ende zum anderen Ende;
einen Schraubteil (16), der an einem Ende des Schaftkörpers (12) vorhanden ist; und
einen Schneideabschnitt (13) zum Schrauben an der Spitze des Schraubteils (16),
**dadurch gekennzeichnet, dass**
der Schneidabschnitt (13) ein Paar spitzer Vorsprünge (20) umfasst, die Schneidklingen (13a) aufweisen, die an Kreuzungsabschnitten zwischen geneigten Ebenen (18, 19) und Kegelflächen (21) am Ende des Schaftkörpers (12) ausgebildet sind.

2. Schraube (11) zum Fixieren eines Atlantoaxialgelenks nach Anspruch 1, wobei:
die paarigen spitzen Vorsprünge (20) einander zugewandt sind und durch Schneiden der Spitze der Schraube (16) in einem geneigten Zustand ausgebildet werden, und die Vorsprünge die Schneidklingen (13a) umfassen.

3. Schraube (11) zum Fixieren eines Atlantoaxialgelenks nach Anspruch 1, wobei:
der Schaftkörper (12) ein Werkzeugeingriffsloch (15) umfasst.

## Revendications

1. Vis destinée à fixer une articulation atloïdo-axoïdienne (11) comprenant :
un corps (12) d'arbre comportant un trou débouchant (17) au centre du corps (12) d'arbre d'une extrémité à l'autre ;
une partie de vissage (16) disposée sur une extrémité du corps (12) d'arbre ; et
une portion coupante (13) destinée au vissage sur la pointe de la partie de vissage (16)
**caractérisée en ce que**,
la portion coupante (13) comprend une paire de saillies acérées (20) ayant des lames coupantes (13a) qui sont formées au niveau de portions d'intersection entre des plans inclinés (18, 19) et des faces effilées (21) au niveau de l'extrémité du corps (12) d'arbre.

2. Vis (11) destinée à fixer une articulation atloïdo-axoïdienne selon la revendication 1, dans laquelle :
la paire de saillies acérées (20) sont tournées l'une vers l'autre et sont formées par découpe de la pointe de la vis (16) dans un état d'inclinaison, et les saillies comprennent les lames coupantes (13A).

3. Vis (11) destinée à fixer une articulation atloïdo-axoïdienne selon la revendication 1, dans laquelle :
le corps (12) d'arbre comprend un trou de mise en prise (15) d'outil.
